(19) **Europäisches Patentamt**

**European Patent Office**

**Office européen des brevets**

(11) Veröffentlichungsnummer: **0 331 808 B1**

(12) # EUROPÄISCHE PATENTSCHRIFT

(45) Veröffentlichungstag der Patentschrift:
03.07.91 Patentblatt 91/27

(51) Int. Cl.⁵: **G01N 33/80, G01N 33/556, G01N 33/555**

(21) Anmeldenummer: **88120671.8**

(22) Anmeldetag: **10.12.88**

(54) Immunoassayverfahren zur Blutgruppenbestimmung.

(30) Priorität: **28.01.88 DE 3802452**

(43) Veröffentlichungstag der Anmeldung:
**13.09.89 Patentblatt 89/37**

(45) Bekanntmachung des Hinweises auf die Patenterteilung:
**03.07.91 Patentblatt 91/27**

(84) Benannte Vertragsstaaten:
**AT BE CH DE ES FR GB GR IT LI LU NL SE**

(56) Entgegenhaltungen:
**EP-A- 0 084 102**
**EP-A- 0 130 434**
**EP-A- 0 223 978**

(56) Entgegenhaltungen:
**WO-A-87/07304**
**US-A- 4 328 183**
**B.D. DAVIS et al.: "Microbiology", Edition 3, Teil "Immunology", 1980, Seiten 290-548, Harper & Row, New York, US**

(73) Patentinhaber: **Biotest AG**
**Flughafenstrasse 4**
**W-6000 Frankfurt am Main 73 (DE)**

(72) Erfinder: **Uthemann, Horst, Dr. Dipl.-Chem.**
**Sachsenhäuser Landwehrweg 66**
**W-6000 Frankfurt am Main 70 (DE)**

(74) Vertreter: **Beil, Hans Christoph, Dr. et al**
**Beil, Wolff und Beil Rechtsanwälte**
**Adelonstrasse 58**
**W-6230 Frankfurt am Main 80 (DE)**

Jouve, 18, rue Saint-Denis, 75001 PARIS

**Beschreibung**

Die Erfindung betrifft ein Verfahren zur Blutgruppenbestimmung gemäss dem Oberbegriff des Anspruchs 1.

In den letzten Jahrzehnten stellte die Agglutinationsreaktion aufgrund ihrer Einfachheit und Vielseitigkeit eine der gebräuchlichsten Methoden zur Blutgruppenbestimmung dar. Einer ihrer wesentlichen Nachteile besteht jedoch im Fehlen eines objektiven Endpunktes, der leicht automatisch oder auch visuell bestimmt werden kann, da innerhalb der Verdünnungsreihen unterschiedlich starke bzw. schwache Reaktionen erfolgen, wobei z.B. schwache Reaktionen als falsch negativ angesehen werden können.

Die EP-A 0 084 102 offenbart beispielsweise gebrauchsfertige, mit angetrockneten Antiseren beschichtete Mikrotiterplatten, mit denen Agglutinationsreaktionen zur Blutgruppenbestimmung durchgeführt werden und weist daher ebenfalls die vorgeschilderten Schwierigkeiten auf.

In jüngerer Zeit fand der ELISA-Test (enzyme-linked immunosorbent assay) zur Blutgruppenbestimmung Anwendung, der zwar eine ausgezeichnete Empfindlichkeit aufweist, dessen Nachteile jedoch in der Vielzahl der Handhabungsstufen, den Problemen bezüglich der Antikörper/Enzym-Absorbierbarkeit durch Erythrozyten und der Wechselwirkung von interzellularem Hämoglobin in Lösung liegen.

Als Alternative zu Agglutinationsreaktionen und zum ELISA-Test wurden in jüngerer Zeit Festphasen-Methoden zur Blutgruppenbestimmung im ABO- und Rh-System, zur Antikörpersuche und zur Kreuzprobe beschrieben (H.H.Moore et al, Transfusion 22 1982 : 540 (1) ; H.H.Moore, Transfusion 24 1984 : 218-221 (2) ; L.T.Sinor et al., Transfusion 25 1985 : 21-23 (3); F.V.Plapp et al, A.J.C.P. 82 1984 : 719-721 (4) ; M.L.Beck et al, Med.Lab.Sci. 41 1984 : 374-381 (5) ; J.M.Rachel et al ; 25 1985 : 24-26 (6) ; F.V.Plapp et al, The Lancet, 1986 : 1465-1466 (7)). Diesen Methoden ist gemeinsam, dass sich positive Reaktionsmuster als Schicht spezifisch gebundener Erythrozyten an speziell präparierten festen Phasen darstellen, während negative Reaktionen nicht zur Ausbildung einer Festphasenschicht führen. Die unterschiedlichen Reaktionsmuster lassen sich visuell oder spektrophotometrisch ablesen, und man kommt zu einer objektiven Ja- oder Nein-Bestimmung.

Festphasen-Methoden zur ABO- und Rh-Bestimmung wurden bisher so ausgeführt, dass geeignetes Trägermaterial wie z.B. Mikrotiterplatten aus Polyvinylchlorid (2) oder Polystyrol (3) oder Membranen aus Nylon oder Nitrocellulose (7) Pufferlösungen monoklonaler oder affinitätsgereinigter Antikörper längere Zeit, z.B. mindestens 2 Stunden (2), zur Beschichtung ausgesetzt wurde, wobei es zur Ausbildung immobilisierter, d.h. irreversibel gebundener monomolekularer Festphasen-Schichten auf den Kunststoffoberflächen durch passive Adsorption kam. Überschüssige Antikörper wurden weggewaschen, und die auf diese Weise vor der Verwendung präparierten Träger standen dann für blutgruppenserologische Untersuchungen zur Verfügung. Die Verwendung von Anti-D vom IgG-Typ für die Rh-Bestimmung erforderte die Ausbildung einer Festphasen-Doppelschicht, bestehend aus affinitätsgereinigtem Anti-Human-IgG (3) + (5) und Anti-D, um den Abstand zwischen Kunststoffoberfläche und angebotenen Erythrozyten zu überwinden.

Obgleich die bisher bekannten Festphasen-Methoden gegenüber der Agglutinationsmethode und der ELISA-Methode beachtliche Vorteile aufweisen, empfand man die Vorbeschichtung der Trägeroberfläche immer noch als zu aufwendig.

Der Erfindung liegt die Aufgabe zugrunde, ein vereinfachtes und weniger aufwendiges Verfahren zur Blutgruppenbestimmung mit Hilfe der Festphasen-Methode bereitzustellen.

Diese Aufgabe wird erfindungsgemäss durch die kennzeichnenden Merkmale des Anspruchs 1 gelöst.

Überraschenderweise wurde gefunden, dass auf eine mehr oder weniger aufwendige Vorbeschichtung der Trägeroberfläche verzichtet werden kann, wenn es sich bei den Antikörpern um spezifisch reagierende Immunglobuline vom IgM-Typ handelt, weil es gleichzeitig zur Ausbildung von Festphasen-Schichten und spezifischer immunologischer Bindung von Erythrozyten auf der Trägeroberfläche kommt, wenn in einem Schritt der Trägeroberfläche die Antikörper vom IgM-Typ und Erythrozytensuspensionen angeboten werden. Vorteilhaft aber nicht Bedingung ist die Verwendung monoklonaler oder affinitätschromatographisch gereinigter Antikörper.

Besonders überrascht die kurze Zeit, in der es in ausreichender Menge zur Bindung von Antikörpern an die Oberflächen kommt. Bei Verwendung von Antikörperkonzentrationen, die auf die Größe der jeweiligen Trägeroberfläche so abgestimmt werden, dass sie nicht höher sind, als erforderlich ist, um auf der Trägeroberfläche eine irreversible bzw. immobilisierte Schicht der Antikörpermoleküle zu bilden, d.h. die nicht mehr ausreichen, um eine Agglutinationsreaktion herbeizuführen, bildet sich bei entsprechender Spezifität die Zelladhäsion nach Zugabe der Erythrozytensuspensionen sofort bei Zentrifugation des Trägerkörpers oder innerhalb von 30-40 Minuten beim Stehen bei Raumtemperatur aus.

In den nachstehenden Versuchen wurden als Träger Mikrotiterplatten aus Polystyrol verwendet, obgleich jede andere Art von Träger bzw. Trägermaterial, wie sie bei den bekannten Verfahren gemäss (1) bis (7) beschrieben wird, verwendbar sein kann. Mit Bezug auf die Näpfchengrösse handelsüblicher Mikrotiterplatten erwiesen sich Verdünnungen von Antikörpern in isotoner Kochsalzlösung im Verhältnis 1 : 10 bis 1 : 1000 in Mengen von 10-50 µl pro Näpfchen als geeignet.

Das erfindungsgemässe Verfahren lässt sich z.B. auf die z.Zt. verfügbaren Antikörper vom IgM-Typ der Spezifitäten Anti-A, Anti-B, Anti-AB, Anti-D, Anti-M, Anti-N, Anti-Le[a] und Anti-Le[b] anwenden.

Als besonders geeignet erwiesen sich monoklonale Antikörper.

Es ist nicht erforderlich, die Antikörper in einer Pufferlösung, wie sie zur Herstellung von Festphasen-Schichten, z.B. von Moore (1, 2), Sinor et al (3), Plapp et al (4) und Beck et al (5) verwendet worden sind, zu verdünnen. Die Erythrozyten werden zweckmässigerweise in Konzentrationen von z.B. 0,3-3,0% ig, vorzugsweise 0,5% ig, in isotoner Kochsalzlösung angesetzt. Vor der Testdurchführung kann eine Enzymbehandlung der zu untersuchenden Erythrozyten erfolgen.

Positive Reaktionen zeigen einen deutlich ausgebildeten Zellrasen durch spezifische Bindung der Erythrozyten über die angebotenen Antikörper an die Wandungen der Näpfchen der Mikrotiterplatten, während man negative Reaktionen an Knöpfchen auf den Näpfchenböden sedimentierter Zellen erkennt. Die unterschiedlichen Reaktionsmuster können visuell beurteilt oder mit einem Spektrophotometer-System, beispielsweise einem Photometer-Computer-System automatisch gelesen und interpretiert werden.

## Beispiel 1

Verdünnungen monoklonaler Antikörper vom IgM-Typ mit den Spezifitäten Anti-A, Anti-B, Anti-AB und Anti-D in isotoner Kochsalzlösung im Verhältnis 1 : 10 bis 1 : 1000 (je 50 µl) wurden mit 0,5% igen Erythrozytensuspensionen in Kochsalz (je 50 µl) in je ein Näpfchen (U-Form) einer Mikrotiterplatte aus Polystyrol pipettiert. Nach ausreichender Durchmischung (Schüttelgerät) lieb man die Mikrotiterplatte bei Raumtemperatur 30 Minuten stehen. Danach konnte visuell oder automatisch ausgewertet werden.

Positive Reaktionen zeigten einen Zellrasen auf der Näpfchenoberfläche, während Knöpfchen sedimentierter Zellen auf den Näpfchenböden negative Ergebnisse darstellten.

## Beispiel 2

Nach Zugabe der verdünnten Antikörper und der Erythrozytensuspensionen und deren Durchmischung wie in Beispiel 1 beschrieben, wurden die Mikrotiterplatten sofort z.B. 1 Minute bei 1000 U/Minute zentrifugiert, und es wurde wie bereits beschrieben ausgewertet.

Ergebnisse :

Nach den beschriebenen Verfahren gemäss den Beispielen 1 und 2 wurden im Vergleich zu Agglutinationsreaktionen 864 Bestimmungen mit übereinstimmenden Ergebnissen durchgeführt. Das neue Verfahren ist empfindlich, leicht und auch halbautomatisch durchzuführen. D[u]-Blute wurden mit diesen Methoden nicht erfasst.

## Ansprüche

1. Verfahren zur Blutgruppenbestimmung mit Hilfe der Festphasen-Methode unter Verwendung blutgruppenspezifischer Antikörper vom IgM-Typ und eines Trägers, an dessen Oberfläche sich die Antikörper irreversibel binden lassen, dadurch gekennzeichnet, dass man eine Lösung des Antikörpers vom IgM-Typ gleichzeitig mit der zu bestimmenden Erythrozytensuspension in Kontakt mit der Trägeroberfläche bringt und kurze Zeit nach der Kontaktierung die Bestimmung vornimmt, wobei die Antikörperkonzentration nicht höher sein darf, als erforderlich ist, um auf der Trägeroberfläche eine irreversible bzw. immobilisierte Schicht der Antikörpermoleküle zu bilden, die spezifisch Erythrozyten binden kann, und nicht ausreicht, um eine Agglutinationsreaktion herbeizuführen.

2. Verfahren nach Anspruch 1, dadurch gekennzeichnet, dass man als Antikörper monoklonale oder affinitätsgereinigte polyklonale Antikörper verwendet.

3. Verfahren nach Anspruch 1 oder 2, dadurch gekennzeichnet, dass man als Träger Mikrotiterplatten verwendet.

4. Verfahren nach einem der vorstehenden Ansprüche, dadurch gekennzeichnet, dass man eine isotone Kochsalzlösung der Antikörper im Verhältnis von 1 : 10 bis 1 : 1000 Antikörper zu Lösungsmittel verwendet.

5. Verfahren nach den Ansprüchen 3 und 4, dadurch gekennzeichnet, dass man 10-50 µl der Antikörperlösung pro Näpfchen einer Mikrotiterplatte zugibt. lösung pro Näpfchen einer Mikrotiterplatte zugibt.

6. Verfahren nach einem der vorstehenden Ansprüche, dadurch gekennzeichnet, dass man als Trägermaterial Polystyrol verwendet.

7. Verfahren nach einem der vorstehenden Ansprüche, dadurch gekennzeichnet, dass man eine 0,3 bis 3,0% ige, vorzugsweise 0,5% ige isotone Kochsalzlösung der zu bestimmenden Erythrozyten verwendet.

8. Verfahren nach einem der vorstehenden Ansprüche, dadurch gekennzeichnet, dass man die Bestimmung visuell oder spektrophotometrisch vornimmt.

## Claims

1. A process for determining blood groups by the solid-phase method using blood-group-specific antibodies of the IgM type and a support to the surface of which the antibodies can be irreversibly fixed, characterized in that a solution of the antibody of the IgM type is brought into contact with the surface of the support simultaneously with the erythrocyte suspension to be determined and the determination is carried out shortly after contacting, the antibody concentration having to be no higher than is necessary to form on the surface of the support an irreversible or immobilized layer of the antibody molecules which can specifically bind erythrocytes and is not sufficient to produce an agglutination reaction.

2. A process as claimed in Claim 1, characterized in that monoclonal or affinity-purified polyclonal antibodies are used as the antibodies.

3. A process as claimed in claim 1 or 2, characterized in that microtiter plates are used as the supports.

4. A process as claimed in any of the preceding claims, characterized in that an isotonic sodium chloride solution of the antibodies in a ratio of 1 : 10 to 1 : 1000 antibodies to solvent is used.

5. A process as claimed in claims 3 and 4, characterized in that 10 to 50 µl of the antibody solution per cell of a microtiter plate are added.

6. A process as claimed in any of the preceding claims, characterized in that polystyrene is used as the support material.

7. A process as claimed in any of the preceding claims, characterized in that a 0.3 to 3.0% and preferably 0.5% isotonic sodium chloride solution of the erythrocytes to be determined is used.

8. A process as claimed in any of the preceding claims, characterized in that the determination is conducted visually or using a spectrophotometer.

## Revendications

1. Procédé de détermination des groupes sanguins à l'aide d'une méthode en phase solide en utilisant des anticorps du type IgM spécifiques des groupes sanguins et un support à la surface duquel les anticorps se lient d'une manière irréversible, caractérisé en ce qu'on amène une solution de l'anticorps du type IgM, en même temps que la suspension d'érythrocytes à déterminer, en contact avec la surface du support, et en ce qu'on effectue la détermination peu de temps après la mise en contact, la concentration des anticorps ne devant pas être plus élevée qu'il n'est nécessaire pour former à la surface du support une couche irréversible ou immobilisée des molécules d'anticorps, qui peut lier spécifiquement les érythrocytes, et qui n'est pas suffisante pour provoquer une réaction d'agglutination.

2. Procédé selon la revendication 1, caractérisé en ce qu'on utilise comme anticorps, des anticorps monoclonaux ou polyclonaux purifiés par affinité.

3. Procédé selon la revendication 1 ou 2, caractérisé en ce qu'on utilise comme support une plaque de microtitrage.

4. Procédé selon l'une des revendications précédentes, caractérisé en ce qu'on utilise une solution des anticorps dans le chlorure de sodium isotonique dans un rapport anticorps : solvant de 1 : 10 à 1 : 1000.

5. Procédé selon les revendications 3 et 4, caractérisé en ce qu'on ajoute 10 à 50 µl de la solution d'anticorps par petits godets d'une plaque de microtitrage.

6. Procédé selon l'une des revendications précédentes, caractérisé en ce qu'on utilise comme matière de support du polystyrène.

7. Procédé selon l'une des revendications précédentes, caractérisé en ce qu'on utilise une solution des érythrocytes à déterminer dans du chlorure de sodium isotronique à 0,3 à 3,0%, de préférence à 0,5%.

8. Procédé selon l'une des revendications précédentes, caractérisé en ce qu'on effectue la détermination visuellement ou spectrophotométriquement.